# EUROPEAN PATENT SPECIFICATION

(11) **EP 3 372 213 B1**
(45) Date of publication and mention of the grant of the patent: **17.02.2021**
(21) Application number: 16881518.1
(22) Date of filing: 19.10.2016
(51) Int. Cl.: A61F 13/49, A61F 13/496, A61F 13/56

(54) **ABSORBENT ARTICLE**
SAUGFÄHIGER ARTIKEL
ARTICLE ABSORBANT

(30) Priority: 28.12.2015 JP 2015256834
(43) Date of publication of application: 12.09.2018
(73) Proprietor: Unicharm Corporation, Ehime 799-0111 (JP)
(72) Inventor: SAITO, Kyota, Kanonji-shi Kagawa 769-1602 (JP)
(74) Representative: Dolleymores
(86) International application number: PCT/JP2016/080991
(87) International publication number: WO 2017/115538

(56) References cited:
- EP-A1- 2 526 913
- JP-A- 2003 339 772
- JP-A- 2008 104 874
- JP-A- 2008 104 874
- JP-A- 2009 034 209
- JP-A- 2011 147 608
- JP-A- 2012 125 483
- JP-U- H 045 826
- JP-U- H 045 826
- JP-U- H0 631 721

## Description

### [Technical Field]

The present invention relates to an absorbent article.

### [Background Art]

A disposable diaper such as a so-called tape-type diaper or an underpants-shaped disposable diaper is conventionally known as an absorbent article for absorbing excrement. PTL 1 describes a disposable diaper as follow: its front and back waist parts each have one-side (e.g., left) portion and other-side (e.g., right) portion; the one-side (left) portions of the two waist parts are joined to each other, forming a leg hole; the side ends of the other-side (e.g., right) portions remain unjoined; the unjoined portion of the back waist part has a fastening member (joining means) which is capable of forming another leg hole.

### [Citation List]

### [Patent Literature]

[PTL 1] Japanese Unexamined Utility Model Application Publication No. H4-5826

JP 2008 104874 discloses a so-called half-open underpants-shaped diaper.

### [Summary of Invention]

### [Technical Problem]

In a so-called half-open, underpants-shaped diaper described in PTL 1, assuming that the joining strength of a joining portion of front and back waist parts is determined so as to make it easier to pull apart the joining portion of front and back waist parts when changing the diaper; and assuming that the joining strength of the joining portion of the back waist part and the fastening member is set to substantially the same level of the joining strength of the joining portion of front and back waist parts. In this case, there is a possibility that the joining portion of the back waist part and the fastening member is broken by pulling the fastening member when the diaper puts on.

The present invention was achieved in light of the problems described above, and an aspect of the present invention is to provide an absorbent article being a half-open, underpants-shaped diaper whose open side is difficult to break when putting on the diaper and whose side opposite to the open side is easy to be pulled apart when changing the diaper.

### [Solution to Problem]

The present invention provides the absorbent article of independent claim 1. The dependent claims specify preferred but optional features.

Other features of the present invention will become apparent from the description of the present specification and the accompanying drawings.

### [Advantageous Effects of Invention]

According to the invention, it is possible to provide an absorbent article being a half-open, underpants-shaped diaper whose open side is difficult to break when putting on the diaper and whose side opposite to the open side is easy to be pulled apart when changing the diaper.

### [Brief Description of Drawings]

[FIG. 1] FIG. 1 is a schematic perspective view of a half-open, underpants-shaped disposable diaper.
[FIG. 2] FIG. 2 is a plan view of the diaper in an unfolded state.
[FIG. 3] FIG. 3 is a diagram illustrating elastic regions X and Y.
[FIG. 4] FIG. 4A is a schematic cross-sectional view of a front waist portion, FIG. 4B is a schematic cross-sectional view of a back waist portion, and FIG. 4C is a schematic cross-sectional view of a fastening member.
[FIG. 5] FIG. 5A is a diagram showing a first joining portion of Example 1, and FIG. 5B is a diagram showing a second joining portion of Example 2.
[FIG. 6] FIG. 6A is a diagram showing a first joining portion of Example 2, and FIG. 6B is a diagram showing a second joining portion of Example 2.
[FIG. 7] FIGS. 7A to 7C are diagrams showing a second joining portion of Example 3.
[FIG. 8] FIGS. 8A to 8E are diagrams showing a first joining portion of Example 4.
[FIG. 9] FIG. 9A is a side view of a diaper when it is put on, and FIG. 9B is a front view of a diaper when it is put on.
[FIG. 10] FIG. 10A is a schematic plan view showing an example of the position of a fastening portion when a diaper is put on, and FIG. 10B is a schematic top view of a diaper in FIG. 10A.

### [Description of Embodiments]

At least the following matters will be made clear by the description in the present specification and the accompanying drawings.

An absorbent article having a longitudinal direction, a lateral direction intersecting the longitudinal direction, and a front-back direction intersecting the longitudinal direction and the lateral direction, the absorbent article including: a front waist portion extending along the lateral direction; a back waist portion extending along the lateral direction; and a crotch portion provided between the front waist portion and the back waist portion, a one-side end portion of the back waist portion on a one side in the lateral direction being joined by a first joining portion to a one-side end portion of the front waist portion on the one side in the lateral direction, an other-side end portion of the back waist portion on an other side in the lateral direction being joined by a second joining portion to a fastening member, the fastening member having a fastening portion being capable of being fastened to the front waist portion when putting on the absorbent article, a joining strength of the second joining portion being larger than a joining strength of the first joining portion.

With such an absorbent article, the second joining portion (open side) is difficult to break even if the fastening member is pulled when putting on the diaper. Also, the first joining portion (the side opposite to the open side) is easy to be pulled apart when changing the diaper, making it easier to change the diaper 1.

In such an absorbent article, it is desirable
that each of the first joining portion and the second joining portion has a plurality of welded regions, and
that a proportion of an area of the plurality of welded regions of the second joining portion to an area of an entire region of the second joining portion is larger than a proportion of an area of the plurality of welded regions of the first joining portion to an area of an entire region of the first joining portion.

With such an absorbent article, it is possible to make a joining strength of the second joining portion larger than a joining strength of the first joining portion.

In such an absorbent article, it is desirable that a pattern of the plurality of welded regions in the first joining portion is different from a pattern of the plurality of welded regions in the second joining portion.

With such an absorbent article, it is possible to make larger the proportion of the area of the welded regions to the area of the entire region of the second joining portion than the proportion of the area of the welded regions of the first joining portion to the area of the entire region of the first joining portion. This can make it more difficult for the second joining portion to break, and also can make it easier for the first joining portion to be pulled apart.

In such an absorbent article, it is desirable that a laterally inward end of each welded region of the second joining portion has a predetermined length along the longitudinal direction.

With such an absorbent article, even if, when putting on the absorbent article, the fastening member is pulled in the lateral direction and a force is exerted on the laterally inward end of the welded region of the second joining portion, the laterally inward end is less likely to be the origin of breakage. This can make it more difficult for the second joining portion to break.

In such an absorbent article, it is desirable that a lateral length of each welded region of the first joining portion is smallest in at least either of a longitudinal upper end portion of the welded region and a longitudinal lower end portion of the welded region.

With such an absorbent article, the strength of the welded region of the first joining portion is smallest in the upper end portion and the lower end portion. Accordingly, when longitudinal force is exerted to break the first joining portion for changing the absorbent article, the upper end portion and lower end portion of the welded region become the origin of pulling apart. This can make it easier for the first joining portion to be pulled apart.

In such an absorbent article, it is desirable that, while the fastening portion being fastened to the front waist portion so that an end of the fastening portion on a side closer to the second joining portion is positioned in the lateral direction on an end of the crotch portion opposite to the first joining portion, the second joining portion is located on a front side relative to the first joining portion.

With such an absorbent article, the second joining portion has a high rigidity because of being formed by joining the back waist portion and the fastening member, and the second joining portion is located on the front side (stomach side) of the wearer. This makes it possible to suppress downward positional shift of the absorbent article (front waist portion) caused by the protrusion of the wearer's stomach.

In such an absorbent article, it is desirable that a rigidity in the second joining portion is larger than a rigidity in the front waist portion.

With such an absorbent article, this further ensures support for downward positional shift of the front waist portion, further suppressing downward positional shift of the absorbent article (the front waist portion) caused by the protrusion of the wearer's stomach.

In such an absorbent article, it is desirable that a number of components stacked in the second joining portion is larger than a number of components stacked in the front waist portion.

With such an absorbent article, it is possible to make the rigidity in the second joining portion larger than the rigidity in the front waist portion.

The following describes a half-open underpants-shaped disposable diaper mainly for use of infants as an example of an absorbent article according to the present embodiment.

### Basic Configuration of Half-Open Underpants-shaped Disposable Diaper 1

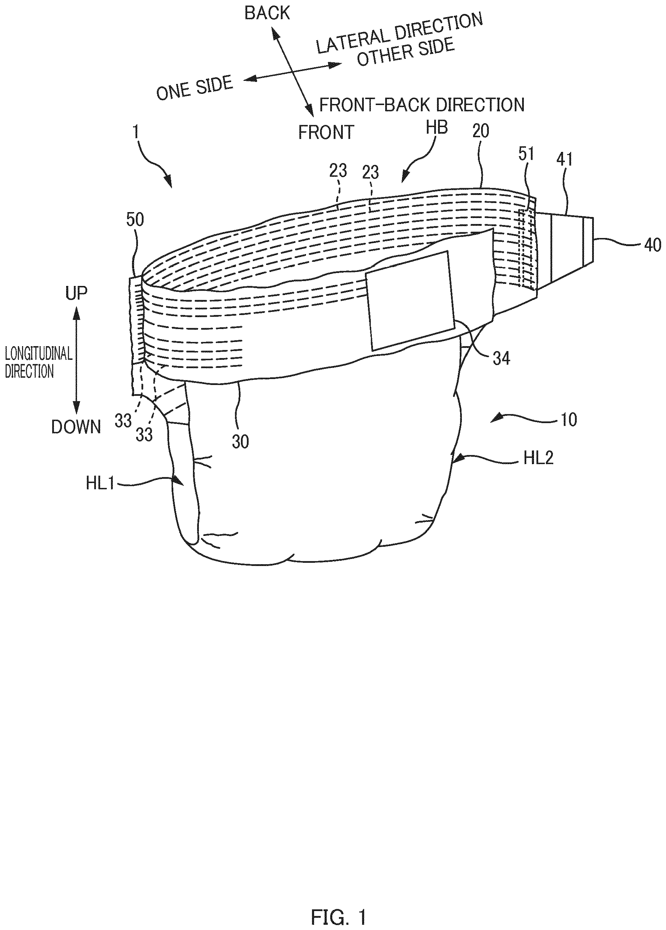

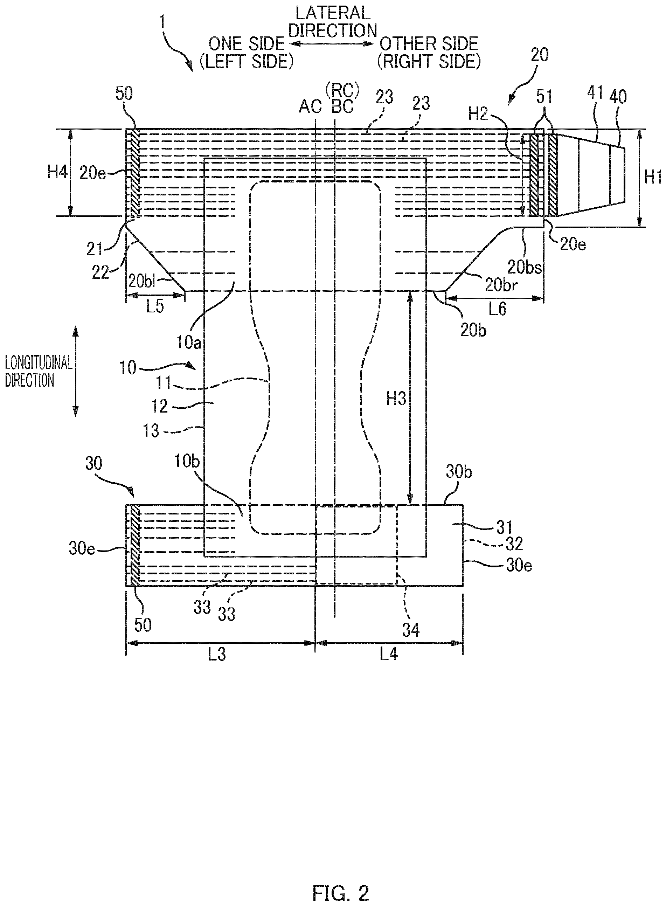

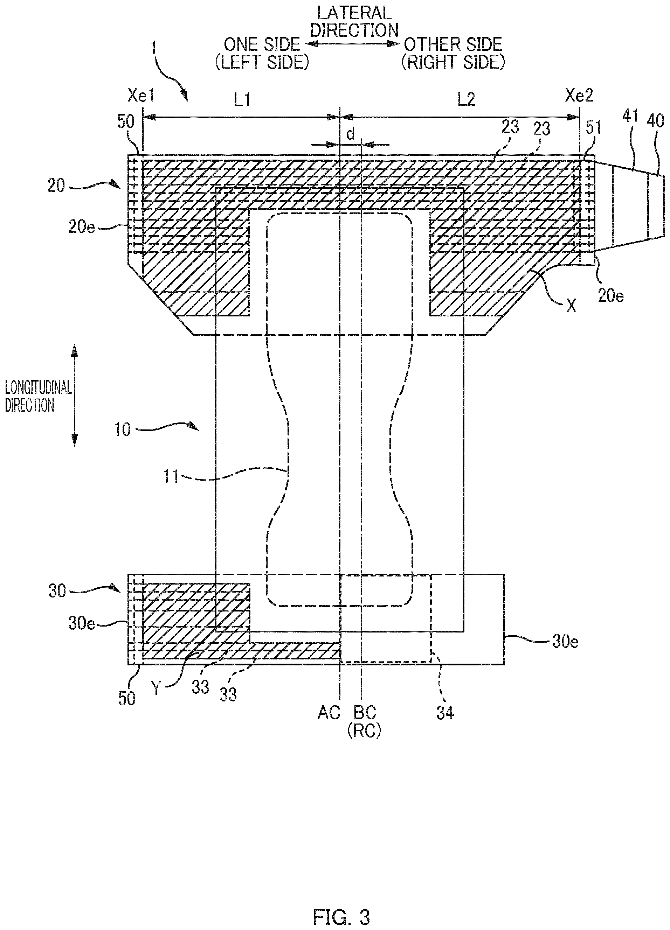

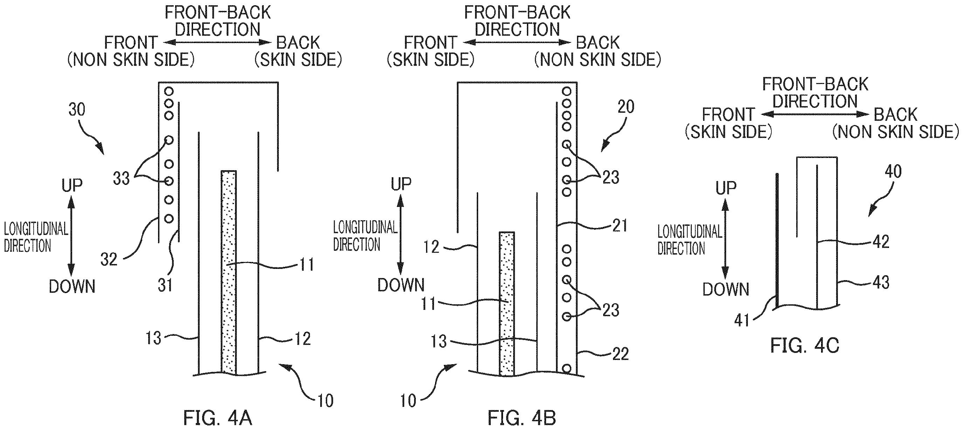

FIG. 1 is a schematic perspective view of a half-open, underpants-shaped disposable diaper 1 (hereinafter referred to as a diaper). FIG. 2 is a plan view of the diaper 1 in an unfolded state; It is a diagram of the diaper 1 whose elastic members are stretched to the level that creases of the diaper 1 disappear. FIG. 3 is a diagram illustrating elastic regions X and Y. FIG. 4A is a schematic cross-sectional view of a front waist portion 30, FIG. 4B is a schematic cross-sectional view of a back waist portion 20, and FIG. 4C is a schematic cross-sectional view of a fastening member 40.

The diaper 1 has a "longitudinal direction", a "lateral direction" that intersects the longitudinal direction, and a "front-back direction" that intersects the longitudinal direction and the lateral direction. And, the diaper 1 includes: "an absorbent main body 10 (a crotch portion)" that is arranged at the crotch of the wearer and absorbs excrement; "a back waist portion 20" that covers the back side of the wearer; a "front waist portion 30" that covers the stomach side of the wearer; and a "fastening member 40" projecting laterally outward from an end portion of the back waist portion 20 on the lateral other side. In FIGS. 2 and. 3, a center line RC indicates the lateral center of the back waist portion 20, a center line BC indicates the approximate center of the body of a wearer when a wearer' s leg has been inserted, and a center line AC indicates the lateral center of the absorbent main body 10. The center line RC is at the same position as the center line BC, and the center lines RC and BC are located on the other side (right) in the lateral direction relative to the center line AC.

The unfolded state shown in FIG. 2 is a state where a first joining portion 50 and a second joining portion 51 in FIG. 1 is uncoupled and opened and the fastening member 40 is detached from the front waist portion 30, the entirety of the diaper 1 is laid flat. In unfolded state shown in FIG. 2, the lengthwise directions of the back waist portion 20 and the front waist portion 30 extends along the lateral direction of a diaper 1, and the back waist portion 20 and the front waist portion 30 are arranged longitudinally spaced apart. Between the back and front waist portions 20 and 30, the absorbent main body 10 (a crotch portion) is disposed with its lengthwise direction extending along the longitudinal direction of the diaper 1. The back waist portion 20 is fixed to the skin-side surface of the lengthwise one end portion of the absorbent main body 10, and the front waist portion 30 is fixed to the skin-side surface of the lengthwise other end portion of the absorbent main body 10.

The diaper 1 in the unfolded state shown in FIG. 2 is folded in half with a substantial center in the longitudinal direction. And, on the lateral one side of the diaper 1, end portions of the back waist portion 20 and the front waist portion 30 are joined together in the first joining portion 50. Consequently, a leg opening HL1 on the one side is formed.

On the lateral other side of the diaper 1, end portions of the back waist portion 20 and the front waist portion 30 are not joined together, but the lateral other-side end portion of the back waist portion 20 and the fastening member 40 are joined in the second joining portion 51. A leg opening HL2 on the other side and a waist opening HB are formed by fastening the fastening member 40 to the front waist portion 30.

A method for putting on such a half-open underpants-shaped diaper 1 is as follow: first, an operator has the wearer's leg (a right leg) insert into a leg opening HL1 on the one side; and then, the operator places the wearer's other leg (a left leg) in a leg opening HL2 on the other side, and pulls the fastening member 40 by his/her one hand while holding the lateral other-side end portion of the front waist portion 30 on the other hand; and finally fastens the fastening member 40 to the front waist portion 30. Accordingly, unlike putting on a so-called pull-on diaper, it is not necessary to insert both legs into the leg openings at once. This makes it possible to easily put the diaper 1 on an infant even if the infant is wriggling their legs.

### Absorbent Main Body 10

The absorbent main body 10 (a crotch portion) is approximately shaped as an elongated rectangle in a plan view, and includes: an absorbent body 11 that absorbs and holds a liquid; a liquid-permeable top face sheet 12 (e.g., nonwoven fabric) that covers the absorbent body 11 on the wearer's skin side; and a liquid-impermeable back face sheet 13 that covers the absorbent body 11 on the non-skin side (e.g., polyethylene film, polypropylene film). The absorbent body 11 is constituted by liquid-absorbent fibers such as pulp fibers and is formed with a predetermined shape such as approximately an hourglass shape, and has a superabsorbent polymer incorporated therein. Though not shown in the drawings, the absorbent main body 10 may include barrier cuff portions preventing side leakage, and leg gather portions improving a fit around legs.

### Back Waist Portion 20

The back waist portion 20 has a planar shape formed by combining a rectangle and a substantial trapezoid. The back waist portion 20 includes: a skin-side sheet 21 that is located on the wearer's skin side (e.g., a flexible sheet such as nonwoven fabric); a non-skin-side sheet 22 that is located on the non-skin side (e.g., a flexible sheet such as nonwoven fabric); and a plurality of elastic strings 23 that are located between the skin-side sheet 21 and the non-skin-side sheet 22. As shown in FIG. 4B, longitudinal upper end portion of the non-skin-side sheet 22 is folded back toward the skin side so as to wrap the upper end portion of the skin-side sheet 21 and the upper end portion of the absorbent main body 10. This reduces stress around the wearer's waist caused when putting on the absorbent article.

As shown in FIG. 2, the back waist portion 20 has an inclined portion 20bl in its lower end portion on the lateral one side, and the inclined portion 20bl is inclined longitudinally obliquely downward from the lateral one-side end 20e toward the other side. Also, in the lower end portion of the back waist portion 20, the following portions are provided on the lateral other side: a straight portion 20bs being substantially parallel with the lower end 30b of the front waist portion 30; and an inclined portion 20br being symmetrical with the inclined portion 20bl about the center line AC. The straight portion 20bs being substantially parallel with the lower end of the front waist portion 30, when a fastening portion 41 is fastened to the front waist portion 30, improves the appearance of the diaper 1 while being put on. It is preferable that the lateral length L6 of the straight portion 20bs and the inclined portion 20br is larger than the lateral length L5 of the inclined portion 20bl (L6 > L5). This makes it possible for the one-side leg opening HL1 to fit a wearer's leg by setting the length of the one-side leg opening HL1 according to an estimated circumference of a wearer's leg, and also makes it possible to enlarge the adjustable range for the sizes of the other-side leg opening HL2 and the waist opening HB, which is adjustable by fastening position of the fastening member 40.

The elastic strings 23 are elastic members that give the back waist portion 20 lateral stretchability. A plurality of the elastic strings 23 are arranged side-by-side at a predetermined longitudinal interval, extending along the lateral direction from the lateral end 20e of the back waist portion 20 on the one side to the lateral end 20e on the other side. However, the elastic strings 23 which overlap the absorbent body 11 in the longitudinal direction have discontinuous parts on the absorbent body 11. In the first joining portion 50 and the second joining portion 51, the elastic strings 23 have no substantial stretching force. Accordingly, the hatched region in FIG. 3 is an elastic region X in which the stretching force of the elastic strings 23 is produced. The lateral one-side end Xe1 of the elastic region X is aligned with the lateral end of the first joining portion 50 on the other side (inside), and the other-side end Xe2 of the elastic region X is aligned with the lateral end of the second joining portion 51 on the one side (inside).

The distance L1 from the lateral one-side end Xe1 of the elastic region X to the center line AC is smaller than the distance L2 from the lateral other-side end Xe2 of the elastic region X to the center line AC (L1 < L2). This makes it possible to suppress the absorbent main body 10 from shifting to the lateral other side due to the fastening member 40 being pulled when the diaper is put on, and consequently it is possible to suppress the absorbent main body 10 from being positioned away from the substantial center of the wearer's body. That is, in the diaper 1, when the wearer's one leg is inserted, the center line AC of the absorbent main body 10 is located on the one side in the lateral direction relative to substantially the center line BC of the wearer's body. Accordingly, when fastening the fastening member 40 to the front waist portion 30 by pulling the fastening member 40 to the lateral other side, the absorbent main body 10 moves toward the lateral other side, the center (AC) of the absorbent main body 10 approaches the center of the wearer's body (BC). This can prevent leakage of excrement, and can reduce discomfort when the diaper is put on. Further, making longer the distance L2 than the distance L1 makes it possible to enlarge the adjustable range for the sizes of the other-side leg opening HL2 and the waist opening HB.

It is preferable that the distance d from the center line RC of the back waist portion 20 to the center line AC of the absorbent main body 10 is smaller than the difference between the distance L2 and the distance L1 (d < L2 - L1). This enables to further reduce a possibility that the center (AC) of the absorbent main body 10 becomes positioned away from the substantial center of the wearer's body (BC).

It is preferable that the length H1 of a lateral end 20e of the back waist portion 20 on the other side is smaller than half the longitudinal length H3 of the absorbent main body 10 from the lower end 20b of the back waist portion 20 to the lower end 30b of the front waist portion 30 (H1 < H3 / 2). Making relatively longer the length H3 of the absorbent main body 10 between the back waist portion 20 and the front waist portion 30 makes it possible for the leg opening HL to have a length according to the size of the legs. In contrast, making shorter the length of the other-side end 20e of the back waist portion 20 makes it easier to transmit to the back waist portion 20 a force for pulling the fastening member 40 when fastening, making it easier to put on the diaper.

### Front Waist Portion 30

The front waist portion 30 has a rectangular shape in a plan view, and includes: a skin-side sheet 31 that is located on the wearer's skin side (e.g., a flexible sheet such as nonwoven fabric); a non-skin-side sheet 32 that is located on the non-skin side (e.g., a flexible sheet such as nonwoven fabric); a plurality of elastic strings 33 that are located between the skin-side sheet 31 and the non-skin-side sheet 32; and the target region 34 on the non-skin-side surface. As shown in FIG. 4A, the longitudinal upper end portion of the non-skin-side sheet 32 is folded back toward the skin side so as to wrap the upper end portion of the skin-side sheet 31 and the upper end portion of the absorbent main body 10. The target region 34 is made of a member capable of engaging with the fastening member 40 (the fastening portion 41), and is provided in a region located on the other side relative to the center line AC of the absorbent main body 10. Note that the lateral length of the front waist portion 30 is smaller than the lateral length of the back waist portion 20.

The elastic strings 33 are elastic members that give the front waist portion 30 lateral stretchability. A plurality of the elastic strings 33 are arranged at longitudinal intervals. And, the elastic strings 33 are provided extending from the center line AC to the lateral end 30e of the front waist portion 30 on the lateral one side, but no elastic string 33 is provided from the center line AC to the lateral end 30e on the lateral other side. In a region in which the front waist portion 30 and the absorbent body 11 is stacked, the elastic string 33 is not provided. As shown in FIG. 3, the elastic region Y is a region in which the elastic strings 33 are provided from the center line AC to an end of the first joining region 50 on the other side (inside) in the lateral direction. Thus, no elastic string 33 is provided in a portion to which the fastening member 40 is fastened and which is located on the other side in the lateral direction relative to the center line AC, and this makes it possible to prevent stretching/contraction of the elastic string 33 from making the fastening more likely to be removed.

It is preferable that the distance L3 from the lateral end 30e of the front waist portion 30 on the lateral one side to the center line AC is larger than the distance L4 from the center line AC to the lateral end 30e of the front waist portion 30 on the lateral other side (L3 > L4). This can make smaller a region of the front waist portion 30 on which the back waist portion 20 is stacked when the fastening member 40 is fastened to the front waist portion 30. This makes it possible to reduce discomfort caused by stacking the back waist portion 20 and the front waist portion 30.

### Fastening Member 40

The fastening member 40 has a substantially trapezoidal shape in a plan view, and includes: a skin-side sheet 42 that is located on the wearer's skin side (e.g., nonwoven fabric); a non-skin-side sheet 43 that is located on the non-skin side (e.g., nonwoven fabric); and the fastening portion 41 capable of engaging with the target region 34. As shown in FIG. 4C, the non-skin-side sheet 43 has a longitudinal, upper end portion which is folded back toward the skin side, and the upper end portion wraps the upper end portion of the skin-side sheet 42. The fastening portion 41 is exemplified by a hook-and-loop fastener having a plurality of fastening projections (hooks); and the target region 34 is exemplified by a hook-and-loop fastener or nonwoven fabric having loops which are to engage with the hooks.

### First Joining Portion 50 and Second Joining Portion 51

As mentioned above, the one-side end portion of the back waist portion 20 on the one side in the lateral direction is joined by the first joining portion 50 to the one-side end portion of the front waist portion 30 on the one side in the lateral direction. And, the other-side end portion of the back waist portion 20 on the other side in the lateral direction is joined by the second joining portion 51 to the fastening member 40, the fastening member 40 having the fastening portion 41 which is capable of being fastened to the front waist portion 30 when the diaper is put on. In the present embodiment, a method for joining of the first joining portion 50 and the second joining portion 51 is welding (e.g., heat welding or ultrasonic welding). And, the first joining portion 50 and the second joining portion 51 each have a plurality of welded regions (depressions). However, a method for joining of the first joining portion 50 and the second joining portion 51 is not limited to welding, and for example, a joining with adhesive may be also employed.

In a half-open, underpants-shaped diaper 1 according to the present embodiment, when changing the diaper, the fastening member 40 is removed from the front waist portion 30 in the lateral other-side end portion. On the other hand, in the lateral one-side end portion, the first joining portion 50 is pulled apart. That is, the second joining portion 51 is not pulled apart. Nevertheless, here, assuming that the second joining portion 51 is configured to have a joining strength so as to be easy to be pulled apart when changing the diaper, like the first joining portion 50. Accordingly, there is a possibility that the second joining portion 51 breaks due to pulling the fastening member 40 in the lateral direction and a direction of the waist circumference of the diaper 1 when putting on the diaper 1. On the contrary, assuming that the joining strength of the first joining portion 50 is made large for preventing breakage of the second joining portion 51. In this case, it is difficult for the first joining portion 50 to be pulled apart when changing the diaper.

In the diaper 1 of the present embodiment, the joining strength of the second joining portion 51 is larger than the joining strength of the first joining portion 50. This makes it difficult for the second joining portion 51 to break even if the fastening member 40 is pulled when putting on the diaper 1. Also, the first joining portion 50 is easy to be pulled apart when changing the diaper 1, making it easier to change diaper 1. Thus, it is preferable that, in a half-open, underpants-shaped diaper 1, the respective joining strengths of the first joining portion 50 and the second joining portion 51 are determined individually according to the their own uses.

If the fastening member 40 is pulled when changing the diaper 1, a force in the lateral direction of the diaper 1 is exerted on the second joining portion 51. And, during a period when putting on the diaper 1, its waist portion is stretched, and therefore a force in the lateral direction of the diaper 1 (a direction of the waist circumference) is exerted on the first joining portion 50 and on the second joining portion 51. Accordingly, in the present embodiment, the joining strengths of the first and second joining portions 50 and 51 are respectively exemplified by load (N) when the first joining portion 50 and the second joining portion 51 are pulled by a tensile tester in the lateral direction of the diaper 1.

A specific method for measuring the joining strength is as follow. First, the pieces of samples 1 and 2 are cut out from a diaper 1 to be measured. The sample 1 includes: the entire region of the first joining portion 50; a part of the back waist portion 20 located on the one side of the first joining portion 50 in the lateral direction of the diaper 1 (a direction of the waist circumference); and a part of the front waist portion 30 on the other side. The sample 2 includes: the entire region of the second joining portion 51; a part of the back waist portion 20 located on the one side of the second joining portion 51 in the lateral direction of the diaper 1 (a direction of the waist circumference); and a part of the fastening member 40 located on the other side.

Next, the sample 1 or 2 is secured the upper and lower chucks of a tensile tester (e.g., Instron Corp., Model 5564). In a case of the sample 1, the back waist portion 20 is fixed to either one of the upper and lower chucks so that the first joining portion 50 is located in the center between the chucks. And, to the other one of the upper and lower chucks, the front waist portion 30 is fixed. In a case of the sample 2, the back waist portion 20 is fixed to either one of the upper and lower chucks so that the second joining portion 51 is located in the center between the chucks. And, to the other one of the upper and lower chucks, the fastening member 40 is fixed.

Then, the upper and lower chucks are separated at a predetermined speed of pulling (e.g., 500 mm/min), pulling the samples 1 and 2. Every time when the distance between the chucks increases by a certain distance (e.g., 10 mm), the current load (N) is obtained. Regarding the samples 1 and 2, n number (e.g., 10) of each samples are measured, the average among number n of the load (N) is calculated. The average load (N) of the sample 1 is defined as the joining strength of the first joining portion 50, and the average load (N) of the sample 2 is defined as the joining strength of the second joining portion 51, comparing them.

A comparison is not limited to the foregoing comparison between the joining strength of the entire region of the first joining portion 50 and the joining strength of the entire region of the second joining portion 51. For example, a comparison may be made in joining strength per unit width in the longitudinal direction of the diaper 1. In this case, the joining strength (N) which is obtained in the foregoing method for measuring may be converted to a value (e.g., N/25 mm) per unit width in the longitudinal direction (e.g., 25 mm). It is also acceptable that the samples 1 and 2 having a longitudinal length of 25mm are cut out from a diaper 1, measuring the joining strengths of the sample 1 which includes a part of the first joining portion 50 and the sample 2 which includes a part of the second joining portion 51. Or, the load when the first joining portion 50 of the sample 1 (the second joining portion 51 of the sample 2) breaks by increasing the distance between the chucks may be defined as the joining strength of the first joining portion 50 (the second joining portion 51), for example.

Examples of the first joining portion 50 and the second joining portion 51 will be described below.

### Example 1

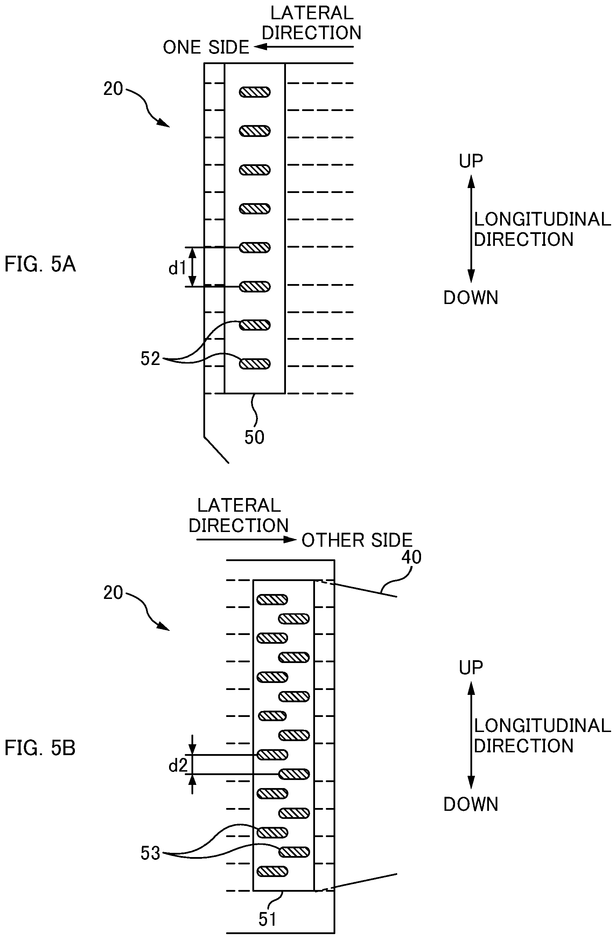

FIG. 5A is a diagram showing the first joining portion 50 of Example 1, and FIG. 5B is a diagram showing the second joining portion 51 of Example 1. In FIG. 5 and FIGS. 6 to 8 (to be describe later), for convenience, the smaller numbers of welded regions 52 and 53 are respectively included in the first joining portion 50 and the second joining portion 51 than the actual number thereof.

The first joining portion 50 of Example 1 includes eight welded regions 52 having a substantially rectangular shape, more specifically, a barrel shape in which the short sides of its rectangle are curved. The eight welded regions 52 are arranged in line side-by-side at a predetermined longitudinal interval d1. On the other hand, the second joining portion 51 includes sixteen welded regions 53, each of which has the same shape and size as the welded region 52 included in the first joining portion 50. The sixteen welded regions53 are arranged laterally in two lines, side-by-side at a predetermined longitudinal interval d2.

As mentioned above, in Example 1, the pattern in the first joining portion 50 composed of a plurality of the welded regions 52 is different from the pattern in the second joining portion 51 composed of a plurality of the welded regions 53. The number of the welded regions 53 included in the second joining portion 51 is larger than the number of the welded regions 52 included in the first joining portion 50. In other words, the longitudinal interval d2 at which the welded regions 53 of the second joining portion 51 are arranged is smaller than the longitudinal interval d1 at which the welded regions 52 of the first joining portion 50 are arranged.

Accordingly, the proportion (A2 / A1) of the area (A2) of a plurality of the welded regions 53 to the area (A1) of the entire region of the second joining portion 51 is larger than the proportion (A4 / A3) of the area (A4) of a plurality of the welded regions 52 to the area (A3) of the entire region of the first joining portion 50. Consequently, the joining strength of the second joining portion 51 is larger than the joining strength of the first joining portion 50. In addition, the second joining portion 51 is difficult to break when putting on the diaper, and the first joining portion 50 is easy to be pulled apart when changing the diaper.

Note that, in the first joining portion 50 and the second joining portion 51, for differing the pattern of the welded regions 52 from the pattern of the welded regions 53, it is preferable that, for example, projections on the outer surface of an embossing roll of a welding apparatus have different patterns. In a case where the number of the welded regions 53 of the second joining portion 51 are large, in order to prevent spaces between adjacent welded regions 53 from excessively narrowing to be the origin of breakage, it is preferable that the welded regions 53 are arranged in two line along the longitudinal direction, or it is preferable that these lines of the welded regions 53 shift in the longitudinal direction.

### Example 2

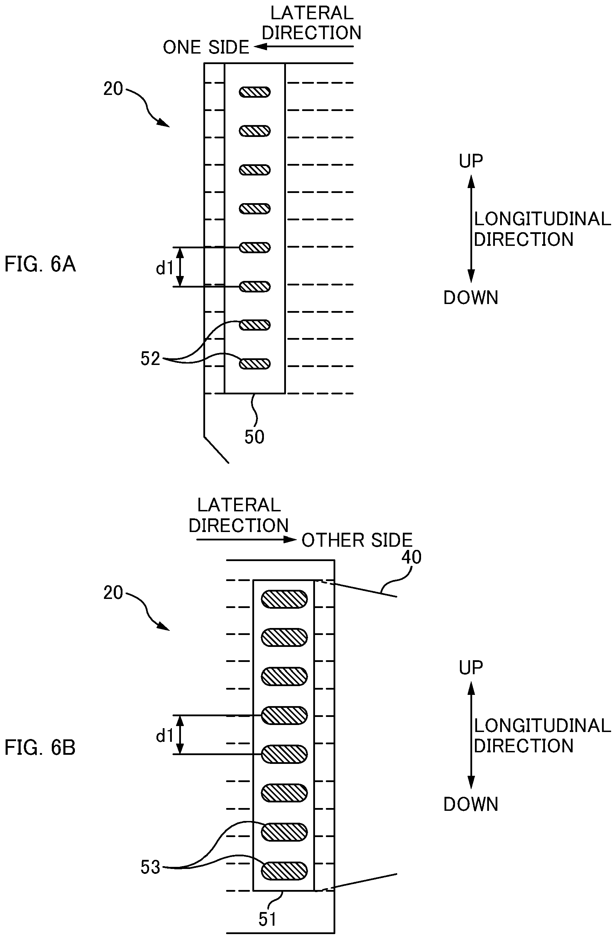

FIG. 6A is a diagram showing a first joining portion of Example 2, and FIG. 6B is a diagram showing a second joining portion of Example 2. In Example 2, the number of the welded regions 52 of the first joining portion 50 is eight the same number of the welded regions 53 of the second joining portion 51. The welded regions 52 and 53 are arranged side-by-side at the same longitudinal interval d1. Each welded region 52 of the first joining portion 50 has the same barrel shape as each welded region 53 of the second joining portion 51, but the welded region 53 of the second joining portion 51 is larger than the welded region 52 of the first joining portion 50.

That is, in Example 2, the pattern in the first joining portion 50 composed of a plurality of the welded regions 52 is different from the pattern in the second joining portion 51 composed of a plurality of the welded regions 53. The size of each welded region 53 of the second joining portion 51 is larger than that of each welded region 52 of the first joining portion 50. Accordingly, the proportion (A2 / A1) of the area (A2) of a plurality of the welded regions 53 to the area (A1) of the entire region of the second joining portion 51 is larger than the proportion (A4 / A3) of the area (A4) of a plurality of the welded regions 52 to the area (A3) of the entire region of the first joining portion 50. Consequently, the joining strength of the second joining portion 51 is larger than the joining strength of the first joining portion 50. In addition, the second joining portion 51 is difficult to break when putting on the diaper, and the first joining portion 50 is easy to be pulled apart when changing the diaper.

Note that, the shapes of the welded regions 52 and 53 in Example 1 and Example 2 are not limited to substantially rectangular shapes. Other shapes such as a polygon, an oval and a star may also be acceptable.

### Example 3

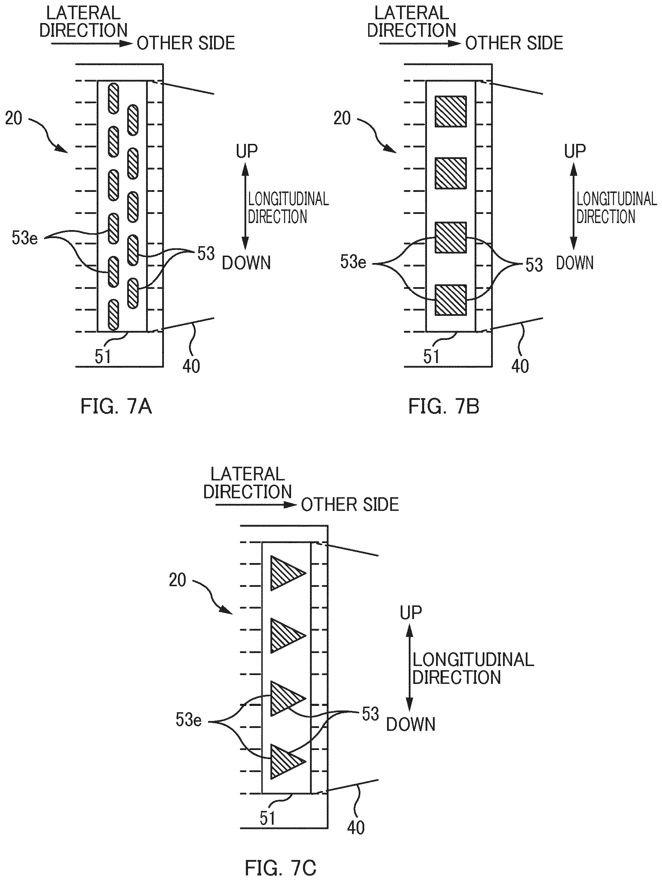

FIGS. 7A to 7C are diagrams showing the second joining portion 51 of Example 3. In FIG. 7A, each welded region 53 of the second joining portion 51 has the same substantially-rectangular (barrel) shape as the welded region 53 of Example 1. But, the orientations thereof are different, and the welded region 53 of Example 3 is arranged with its long sides extending along the longitudinal direction. In FIG. 7B, each welded region 53 has a square shape, and is arranged with two sides extending along the longitudinal direction. In FIG. 7C, each welded region 53 has a triangular shape, and is arranged laterally inside (on the one side), with its one side extending along the longitudinal direction.

If the fastening member 40 is pulled in the lateral direction when the diaper 1 is put on, or if the waist portion stretches during a period when putting on the diaper, a force is exerted on a laterally inward (one-side) end 53e of the welded region 53 included in the second joining portion 51. Accordingly, if the end 53e is for example a vertex of a triangle or a point on an arc, the end 53e works as the origin of breakage. Thus, as in the welded regions 53 shown in FIGS. 7A to 7C, it is preferable that a laterally inward end 53e of each welded region 53 has a certain length along the longitudinal direction. This makes it difficult for the end 53e to be the origin of breakage, making it more difficult for the second joining portion 51 to break.

Note that a state where the laterally inward end 53e of the welded region 53 extends along the longitudinal direction is not limited to a case where the end 53e is parallel to the longitudinal direction, but includes a case where the end 53e intersects the longitudinal direction. Even in the intersecting case, the end 53e is less likely to be the origin of breakage than a case where the end 53e is point shaped, making it difficult for the second joining portion 51 to break. The shapes of the welded regions 53 in Example 3 is not limited to ones shown in FIG. 7. For example, a polygon such as a pentagon, and a semicircle may also be acceptable, and also in this case, the straight portion of each welded region 53 is arranged inside in the lateral direction.

In Example 3, in order to make the joining strength of the second joining portion 51 larger than the joining strength of the first joining portion 50, it is preferable that the proportion of the area of the welded regions 53 in the second joining portion 51 is larger than that of the welded regions 52 in the first joining portion 50. For this purpose, it is preferable that, as in Example 1 or Example 2, the number or the size of the welded regions 52 of the first joining portion 50 is reduced compared to the welded regions 53 of the second joining portion 51. The shape of the welded region 52 of the first joining portion 50 may be different from the shape of the welded region 53 of the second joining portion 51 shown in FIG. 7.

### Example 4

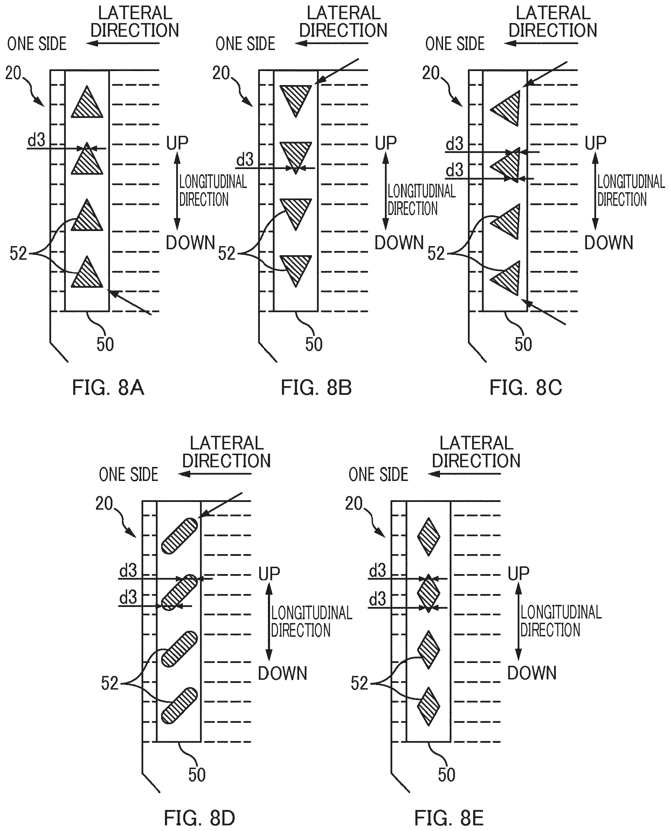

FIGS. 8A to 8E are diagrams showing the first joining portion 50 of Example 4. In FIGS. 8A to 8C, each welded region 52 of the first joining portion 50 has a triangular shape. In FIG. 8A, one vertex of the welded region 52 is located on the upper side in the longitudinal direction. In FIG. 8B, one vertex of the welded region 52 is located on the lower side in the longitudinal direction. In FIG. 8C, one vertex of the welded region 52 is located in a direction extending longitudinally upward and inclined laterally inward (the other side). In FIG. 8D, each welded region 52 has a substantially rectangular (barrel) shape, and the vertexes of the curved short sides are located in a direction extending longitudinally upward and inclined laterally inward. In FIG. 8E, each welded region 52 has a rhomboid shape, and one vertex thereof is on the upper side in the longitudinal direction, one vertex thereof is located on the lower side.

In the welded region 52 of FIG. 8A, the lateral length d3 is smallest in the longitudinal upper end portion of the welded region 52. In the welded region 52 of FIG. 8B, the lateral length d3 is smallest in the longitudinal lower end portion of the welded region 52. In the welded region 52 of FIGS. 8C to 8E, the lateral length d3 is smallest in the longitudinal upper end portion and the longitudinal lower end portion of the welded region 52. That is, in the welded region 52 of the first joining portion 50 in Example 4, the lateral length d3 is smallest in at least either of the longitudinal upper end portion and the longitudinal lower end portion of the welded region 52. Accordingly, in the welded region 52 of the first joining portion 50, the joining strength is smallest in the longitudinal upper end portion and the longitudinal lower end portion. When changing the diaper, the first joining portion 50 is pulled apart along the longitudinal direction, longitudinal force is exerted on the first joining portion 50. Accordingly, concerning the welded region 52 provided in the first joining portion 50, if making smaller the joining strength of its upper and lower end portions, the upper end portion and the lower end portion of the welded region 52 become the origin of pulling apart. This makes it easier to pull apart the first joining portion 50, making it easier to change the diaper 1.

A person who changes the diaper 1, when pulling apart the first joining portion 50, pulls it apart by inserting his/her fingers inside the back waist portion 20 and the front waist portion 30. Accordingly, when he/she starts pulling it apart, the first joining portion 50 is subject to lateral force from inside toward outside in a direction inclined with respect to the longitudinal direction. Consequently, it is preferable that For example like the welded region 52 shown in FIG. 8C, one vertex of the triangle is positioned longitudinally below along a direction inclined laterally outward, and another vertex is positioned longitudinally above along a direction inclined laterally outward. Accordingly, when pulling apart the first joining portion 50 from longitudinally above or below, a vertex of the welded region 52 becomes the origin of pulling apart, making it easier to pull apart the first joining portion 50. In the welded region 52 of FIG. 8A, one vertex of the triangle is positioned longitudinally above along a direction inclined laterally outward, making it easier to pull apart the first joining portion 50 from longitudinally below. In the welded region 52 of FIGS. 8B and 8D, a vertex of the triangle and a vertex of a curved short side are positioned longitudinally below along a direction inclined laterally outward, making it easier to pull apart the first joining portion 50 from longitudinally above.

Also, in Example 4, in order to make the joining strength of the second joining portion 51 larger than the joining strength of the first joining portion 50, it is preferable that the proportion of the area of the welded regions 53 in the second joining portion 51 is larger than that of the welded regions 52 in the first joining portion 50. For this purpose, it is preferable that, as in Example 1 or Example 2, the number or the size of the welded regions 52 of the first joining portion 50 is reduced compared to the welded regions 53 of the second joining portion52. Since it is not necessary to pull apart the second joining portion 51, it is preferable that the welded regions 53 of the second joining portion 51 each have a shape shown in Example 3, not a shape shown in FIG. 8. The arrangement of the welded regions 52 of the first joining portion 50 is not limited to a uniform arrangement along the longitudinal direction. For example, compared to the central portion of the first joining portion 50, the number or the size of the welded regions 52 may be reduced in the upper and lower end portions of the first joining portion 50. This makes smaller the joining strength of the upper and lower end portions of the first joining portion 50, the first joining portion 50 is easier to be pulled apart.

### Others

Note that a method for making the joining strength of the second joining portion 51 larger than the joining strength of the first joining portion 50 is not limited to a method for increasing the proportion of the area of the welded regions 53. For example, the joining strength of each welded region 53 of the second joining portion 51 may be larger than the joining strength of each welded region 52 of the first joining portion 50. Accordingly, it is preferable that, for example, a pressure force applied at a time of welding or a time period of welding increases, making the second joining portion 51 having a larger number of sheets or a larger basis weight of fiber than those of the first joining portion 50.

In the present embodiment, as shown in FIG. 2, the longitudinal length H2 of the second joining portion 51 is smaller than the longitudinal length H4 of the first joining portion 50. However, the present invention is not limited thereto. The length H2 may be equal to the length H4, or the length H2 may be larger than the length H4. Making the length H2 large enables to increase the joining strength of the second joining portion 51.

It is preferable that the longitudinal length H2 of the second joining portion 51 is equal to or larger than half the length H1 of the end 20e of the back waist portion 20 on the lateral other side (H2 > H1 / 2) . Accordingly, when the fastening member 40 is fastened to the front waist portion 30, it is possible to give the force of pulling the fastening member 40 to a not-less-than-half part of the lateral end 20e of the back waist portion 20 on the other side. That is, it is possible to apply to the back waist portion 20 a force which is as evenly-distributed as possible, making it difficult to break the vicinity of the second joining portion 51 when putting on the diaper.

### Position of Second Joining Portion 51 when Diaper is Put On

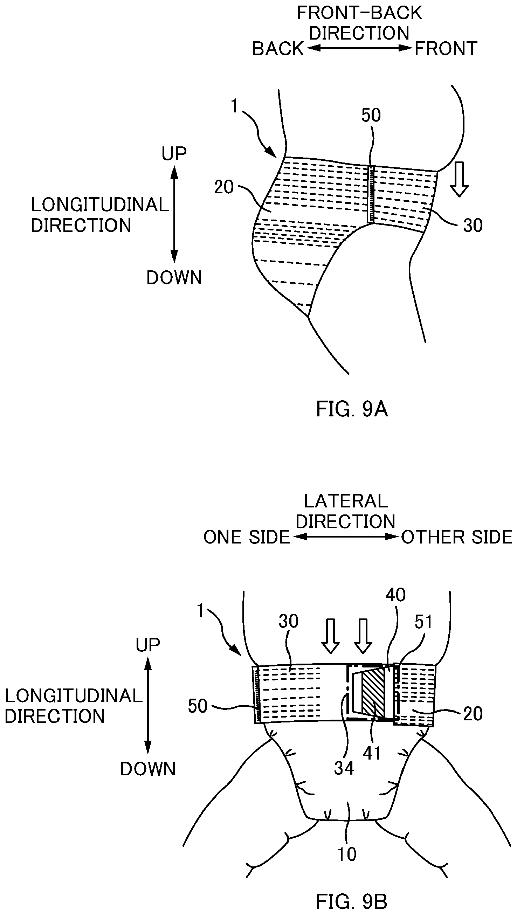

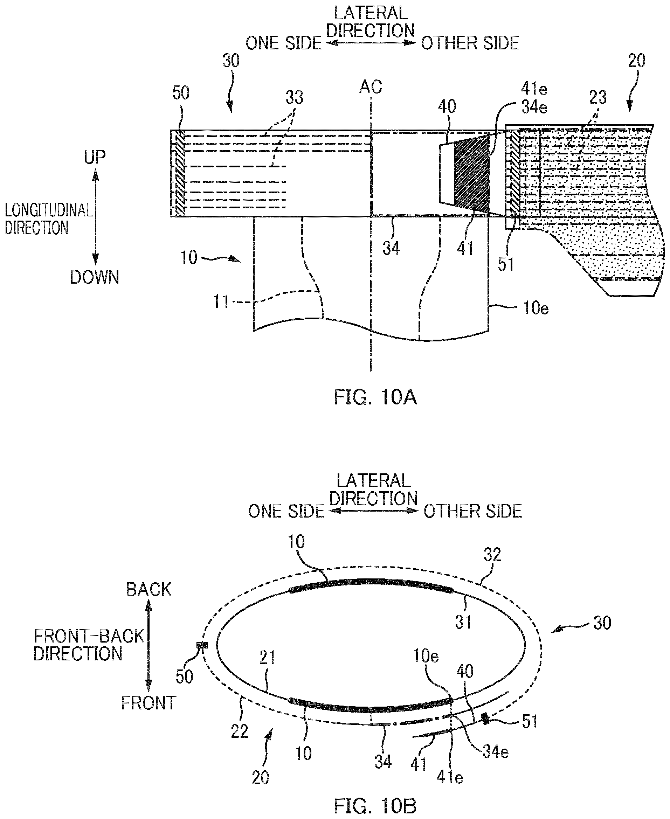

FIG. 9A is a side view of a diaper 1 when it is put on, and FIG. 9B is a front view of a diaper 1 when it is put on. FIG. 10A is a schematic plan view showing an example of the position of the fastening portion 41 when a diaper is put on, and FIG. 10B is a schematic top view of the diaper 1 in FIG. 10A.

In a so-called underpants-shaped diaper, that is a diaper whose back waist portion and front waist portion are joined to each other in the lateral end portions, its joining portions are located on the wearer's sides when the diaper is put on. In the front waist portion, a flexible part made of nonwoven fabric etc. is located on the wearer's front side (the stomach side) . If a wearer of a diaper is an infant, the wearer's stomach usually protrudes forward. Accordingly, in an underpants-shaped diaper, the flexible part of the front waist portion is subject to downward force by the protrusion of the wearer's stomach, making it easier for a diaper to shift downward. This decreases the fit of the absorbent body to the wearer, and produces gaps of leg openings, making it easier to cause leakage of excrement. In addition, the appearance of the diaper when it is put on deteriorates.

However, in a half-open, underpants-shaped diaper 1 according to the present embodiment, the first joining portion 50, which is the joining portion of the back waist portion 20 and the front waist portion 30, is located on one of the wearer's sides when the diaper 1 is put on. On the other hand, the second joining portion 51, which is the joining portion of the back waist portion 20 and the fastening member 40, is located on the front side (stomach side) of the wearer. In other words, as shown in FIG. 10, when the fastening portion 41 is fastened to the front waist portion 30 so that the end 41e (lateral other-side end) of the fastening portion 41 on the side closer to the second joining portion 51 is located in the lateral direction on the end 10e of the absorbent main body 10 (the crotch portion) opposite to the first joining portion 50 (lateral other-side end), the second joining portion 51 is located on the front side relative to the first joining portion 50.

Since in the second joining portion 51, the back waist portion 20 and the fastening member 40 are joined, the second joining portion 51 has a relatively high rigidity. In the diaper 1 according to the present embodiment, the second joining portion 51 having a high rigidity is located on the front side (stomach side) of the wearer. This makes it possible for the second joining portion 51 to support the protrusion of the wearer's stomach. This suppresses downward positional shift of diaper 1 (the front waist portion 30) caused by the protrusion of the wearer's stomach, making better the fit of the diaper 1.

In particular, in the diaper 1 according to the present embodiment, the joining strength of the second joining portion 51 is larger than the joining strength of the first joining portion 50, making larger the rigidity of second joining portion 51. This makes it possible for the second joining portion 51 to securely support the protrusion of the wearer's stomach, making it possible to more securely suppress downward positional shift of the diaper 1 caused by the protrusion of the wearer's stomach.

In the diaper 1, the lateral other-side end 34e of the target region 34 is aligned with the lateral other-side end 10e of the absorbent main body 10. An operator puts the diaper 1 on a wearer so as to place the fastening portion 41 within the target region 34 in order to securely fasten the fastening member 40 to the front waist portion 30. That is, there is a high possibility in which the lateral other-side end 41e of the fastening portion 41 is located laterally inside (on the one side) relative to the lateral other-side end 10e of the absorbent main body 10. If the lateral other-side end 41e of the fastening portion 41 is located at least on the lateral other-side end 10e of the absorbent main body 10 as shown in FIG. 10, the second joining portion 51 is located on the front side relative to the first joining portion 50. Consequently, even if the fastening portion 41 is fastened inside in the lateral direction compared to the case in FIG. 10, it is possible for the second joining portion 51 to be positioned on the front side relative to the first joining portion 50.

In a diaper 1, the rigidity in the second joining portion 51 is larger than the rigidity of the front waist portion 30. This is because, whereas the second joining portion 51 includes the welded region 53 having a high rigidity, the front waist portion 30 is formed of the skin-side sheet 31 and the non-skin-side sheet 32, which are made of such as soft nonwoven fabric. Even if the second joining portion 51 and the front waist portion 30 have an equal rigidity, the second joining portion 51 and the front waist portion 30 in the diaper 1 overlap in the front-back direction (thickness direction) on the wearer's front side. This makes it possible to suppress downward positional shift of the diaper 1, compared to an underpants-shaped diaper which does not include the second joining portion 51 on the wearer's front side. Accordingly, if the rigidity in the second joining portion 51 is larger than the rigidity in the front waist portion 30, this further ensures support for downward positional shift of the front waist portion 30, making it possible to further suppress downward positional shift of the diaper 1 caused by the protrusion of the wearer's stomach.

Note that the values of the rigidities of the second joining portion 51 and the front waist portion 30 are exemplified by a value obtained by dividing a measure value of Gurley stiffness by the length of a sample. The Gurley stiffness is measured using a No. 311 Gurley bending stiffness tester of Yasuda Seiki Seisakusho, LTD., according to JIS-L1096.

In the front waist portion 30, as shown in FIG. 4A, two sheets, the skin-side sheet 31 and the non-skin-side sheet 32, are stacked. Whereas, in the second joining portion 51, as shown in FIGS. 4B and 4C, the following four sheets are stacked in total: the skin-side sheet 21 and the non-skin-side sheet 22 of the back waist portion 20; and the skin-side sheet 42 and the non-skin-side sheet 43 of the fastening member 40. That is, the number of components stacked in the second joining portion 51 is larger than the number of components stacked in the front waist portion 30. Accordingly, the rigidity in the second joining portion 51 is larger than the rigidity in the front waist portion 30. This makes it possible to more securely suppress downward positional shift of the diaper 1 caused by the protrusion of the wearer's stomach. In other words, if the number of components in the second joining portion 51 is larger than the number of components in the front waist portion 30, this can make larger the rigidity in the second joining portion 51 than the rigidity in the front waist portion 30 without taking a measure such as replacing components constituting the second joining portion 51 with thicker members, for example.

In the present embodiment, as shown in FIG. 2, the longitudinal length H2 of the second joining portion 51 is smaller than the longitudinal length H1 of the lateral other-side end portion of the back waist portion 20. But, the length H2 may be equal to the length H1. That is, the second joining portion 51 may be provided from the upper end to the lower end in the lateral other-side end portion of the back waist portion 20. Thus, the longitudinal length of the second joining portion 51 is made as long as possible, making it possible to more securely suppress downward positional shift of the diaper 1 caused by the protrusion of the wearer's stomach.

### Other Embodiments

Although an embodiment of the present invention has been described above, the above embodiment is for facilitating the understanding of the present invention, and is not to be construed as limiting the present invention.

The elastic member included in the diaper 1 is not limited to a string-shaped elastic member such as an elastic string, and may be a sheet-shaped elastic member, for example. In the foregoing embodiment, a diaper having three main components is provided as an example. However, the present invention is not limited thereto. A diaper whose back waist portion 20 and front waist portion 30 are formed as a single unit may be employed, and also a diaper whose back waist portion 20, front waist portion 30 and absorbent main body 10 are formed as a single unit may be employed. In the foregoing embodiment, the diaper 1 is one that is worn by mainly an infant, but the diaper 1 may also be put on an adult such as an elderly. The use of the absorbent article is not limited to a diaper, but the absorbent article may be used as sanitary napkins, for example.

Note that in the above embodiment, the comparison of the lengths H1 to H4, L1 to L6, d, and d1 to d3 shown in FIGS. 2, 3, 5, 6 and 8 is made with stretching the absorbent main body 10, the back waist portion 20, and the front waist portion 30. This "stretching" state is a state where the absorbent main body 10 is stretched in the longitudinal direction without creases, and where the back waist portion 20 and the front waist portion 30 are stretched in the lateral direction without creases. More specifically, it is a state as follow: the absorbent main body 10 is stretched in the longitudinal direction such that the longitudinal dimension thereof reaches a length equal or close to the longitudinal dimension of the top face sheet 12; the back waist portion 20 is stretched in the lateral direction such that the lateral dimension thereof is a length equal or close to the lateral dimension of the skin-side sheet 21 and the lateral dimension of the non-skin-side sheet 22; and the front waist portion 30 is stretched in the lateral direction such that the lateral dimension thereof is a length equal or close to the lateral dimension of the skin-side sheet 31 and the lateral dimension of the non-skin-side sheet 32.

### [Reference Signs List]

1 diaper (absorbent article), 10 absorbent main body (crotch portion), 11 absorbent body, 12 top face sheet, 13 back face sheet, 20 back waist portion, 21 skin-side sheet, 22 non-skin-side sheet, 23 elastic string, 30 front waist portion, 31 skin-side sheet, 32 non-skin-side sheet, 33 elastic string, 34 target region, 40 fastening member, 41 fastening portion, 42 skin-side sheet, 43 non-skin-side sheet, 50 first joining portion, 51 second joining portion, 52 welded region, 53 welded region, HL1 leg opening, HL2 leg opening, HB waist opening, X elastic region, Y elastic region

## Claims

1. An absorbent article (1) having a longitudinal direction, a lateral direction intersecting the longitudinal direction, and a front-back direction intersecting the longitudinal direction and the lateral direction,
the absorbent article comprising:
a front waist portion (30) extending along the lateral direction;
a back waist portion (20) extending along the lateral direction; and
a crotch portion (10) provided between the front waist portion and the back waist portion,
a one-side end portion of the back waist portion (20) on a one side in the lateral direction being joined by a first joining portion (50) to a one-side end portion of the front waist portion (30) on the one side in the lateral direction,
an other-side end portion of the back waist portion (20) on an other side in the lateral direction being joined by a second joining portion (51) to a fastening member (40),
the fastening member (40) having a fastening portion (41) being capable of being fastened to the front waist portion (30) when putting on the absorbent article,
a joining strength of the second joining portion (51) being larger than a joining strength of the first joining portion (50), wherein
an absorbent main body that consists the crotch portion (10) is disposed with its lengthwise direction extending along the longitudinal direction,
at the front waist portion (30), elastic strings (33) are provided from a center line (AC) of the absorbent main body in the lateral direction to an end of the first joining region (50) on the other side in the lateral direction, and
at the front waist portion (30), no elastic string is provided in a portion to which the fastening member (40) is fastened and which is located on the other side in the lateral direction relative to the center line (AC).

2. An absorbent article according to claim 1, wherein
each of the first joining portion (50) and the second joining portion (51) has a plurality of welded regions (52, 53), and
a proportion of an area (A2) of the plurality of welded regions (53) of the second joining portion (51) to an area (A1) of an entire region of the second joining portion (51) is larger than a proportion of an area (A4) of the plurality of welded regions (52) of the first joining portion (50) to an area (A3) of an entire region of the first joining portion (50).

3. An absorbent article according to claim 2, wherein
a pattern of the plurality of welded regions (52) in the first joining portion (50) is different from a pattern of the plurality of welded regions (53) in the second joining portion (51).

4. An absorbent article according to claim 3, wherein
a laterally inward end (53e) of each welded region (53) of the second joining portion (51) has a predetermined length along the longitudinal direction.

5. An absorbent article according to claim 3 or 4, wherein
a lateral length (d3) of each welded region (52) of the first joining portion (50) is smallest in at least either of a longitudinal upper end portion of the welded region (52) and a longitudinal lower end portion of the welded region (52).

6. An absorbent article according to any one of claims 1 to 5, wherein
while the fastening portion (41) being fastened to the front waist portion (30) so that an end of the fastening portion on a side closer to the second joining portion (51) is positioned in the lateral direction on an end of the crotch portion (10) opposite to the first joining portion (50),
the second joining portion (51) is located on a front side relative to the first joining portion (50).

7. An absorbent article according to claim 6, wherein
a rigidity in the second joining portion (51) is larger than a rigidity in the front waist portion (30).

8. An absorbent article according to claim 7, wherein
a number of components stacked in the second joining portion (51) is larger than a number of components stacked in the front waist portion (30).

## Patentansprüche

1. Absorbierender Artikel (1), der Folgendes aufweist: eine Längsrichtung, eine laterale Richtung, die die Längsrichtung schneidet, und eine Vorn-hinten-Richtung, die die Längsrichtung und die laterale Richtung schneidet,
wobei der absorbierende Artikel Folgendes umfasst:
ein vorderes Taillenteil (30), das sich entlang der lateralen Richtung erstreckt;
ein hinteres Taillenteil (20), das sich entlang der lateralen Richtung erstreckt; und
ein Schrittteil (10), das zwischen dem vorderen Taillenteil und dem hinteren Taillenteil bereitgestellt ist,
wobei ein Endteil der einen Seite des hinteren Taillenteils (20) auf einer Seite in der lateralen Richtung durch ein erstes Verbindungsteil (50) mit einem Endteil der einen Seite des vorderen Taillenteils (30) auf der einen Seite in der lateralen Richtung verbunden ist,
ein Endteil der anderen Seite des hinteren Taillenteils (20) auf einer anderen Seite in der lateralen Richtung durch ein zweites Verbindungsteil (51) mit einem Befestigungselement (40) verbunden ist,
das Befestigungselement (40) ein Befestigungsteil (41) aufweist, das an dem vorderen Taillenteil (30) befestigt werden kann, wenn der absorbierende Artikel angelegt wird,
eine Verbindungsstärke des zweiten Verbindungsteils (51) größer ist als eine Verbindungsstärke des ersten Verbindungsteils (50), wobei
ein absorbierender Hauptkörper, der aus dem Schrittteil (10) besteht, mit seiner längsgerichteten Richtung, die sich entlang der Längsrichtung erstreckt, angeordnet ist,
an dem vorderen Taillenteil (30) elastische Bänder (33) von einer Mittellinie (AC) des absorbierenden Hauptkörpers in der lateralen Richtung zu einem Ende des ersten Verbindungsbereichs (50) auf der anderen Seite in der lateralen Richtung bereitgestellt sind und
an dem vorderen Taillenteil (30) in einem Teil, an das das Befestigungselement (40) befestigt wird und das sich auf der anderen Seite in der lateralen Richtung in Bezug auf die Mittellinie (AC) befindet, kein elastisches Band bereitgestellt ist.

2. Absorbierender Artikel nach Anspruch 1, wobei
jedes von dem ersten Verbindungsteil (50) und dem zweiten Verbindungsteil (51) eine Vielzahl von verschweißten Bereichen (52, 53) aufweist und
ein Verhältnis einer Fläche (A2) der Vielzahl von verschweißten Bereichen (53) des zweiten Verbindungsteils (51) zu einer Fläche (A1) eines gesamten Bereichs des zweiten Verbindungsteils (51) größer ist als ein Verhältnis einer Fläche (A4) der Vielzahl von verschweißten Bereichen (52) des ersten Verbindungsteils (50) zu einer Fläche (A3) eines gesamten Bereichs des ersten Verbindungsteils (50).

3. Absorbierender Artikel nach Anspruch 2, wobei
sich ein Muster der Vielzahl von verschweißten Bereichen (52) in dem ersten Verbindungsteil (50) von einem Muster der Vielzahl von verschweißten Bereichen (53) in dem zweiten Verbindungsteil (51) unterscheidet.

4. Absorbierender Artikel nach Anspruch 3, wobei
ein lateral inneres Ende (53e) von jedem verschweißten Bereich (53) des zweiten Verbindungsteils (51) eine vorgegebene Länge entlang der Längsrichtung aufweist.

5. Absorbierender Artikel nach Anspruch 3 oder 4, wobei
eine laterale Länge (d3) von jedem verschweißten Bereich (52) des ersten Verbindungsteils (50) in zumindest einem von einem oberen Endteil in Längsrichtung des verschweißten Bereichs (52) und einem unteren Endteil in Längsrichtung des verschweißten Bereichs (52) am kleinsten ist.

6. Absorbierender Artikel nach einem der Ansprüche 1 bis 5, wobei,
während das Befestigungsteil (41) an dem vorderen Taillenteil (30) derart befestigt ist, dass ein Ende des Befestigungsteils auf einer Seite, die näher zu dem zweiten Verbindungsteil (51) ist, in der lateralen Richtung auf einem Ende des Schrittteils (10) gegenüber des ersten Verbindungsteils (50) positioniert ist,
sich das zweite Verbindungsteil (51) auf einer vorderen Seite in Bezug auf das erste Verbindungsteil (50) befindet.

7. Absorbierender Artikel nach Anspruch 6, wobei
eine Steifigkeit in dem zweiten Verbindungsteil (51) größer ist als eine Steifigkeit in dem vorderen Taillenteil (30).

8. Absorbierender Artikel nach Anspruch 7, wobei
eine Anzahl von Komponenten, die in dem zweiten Verbindungsteil (51) gestapelt sind, größer ist als eine Anzahl von Komponenten, die in dem vorderen Taillenteil (30) gestapelt sind.

## Revendications

1. Article absorbant (1) ayant une direction allant dans le sens longitudinal, une direction allant dans le sens latéral croisant la direction allant dans le sens longitudinal, et une direction allant dans le sens avant-arrière croisant la direction allant dans le sens longitudinal et la direction allant dans le sens latéral,
l'article absorbant comportant :
une partie avant au niveau de la taille (30) s'étendant le long de la direction allant dans le sens latéral ;
une partie arrière au niveau de la taille (20) s'étendant le long de la direction allant dans le sens latéral ; et
une partie au niveau de l'entrejambe (10) mise en œuvre entre la partie avant au niveau de la taille et la partie arrière au niveau de la taille,
une partie d'extrémité d'un côté de la partie arrière au niveau de la taille (20) sur un côté dans la direction allant dans le sens latéral étant assemblée par une première partie d'assemblage (50) à une partie d'extrémité d'un côté de la partie avant au niveau de la taille (30) sur ledit un côté dans la direction allant dans le sens latéral,
une partie d'extrémité d'un autre côté de la partie arrière au niveau de la taille (20) sur un autre côté dans la direction allant dans le sens latéral étant assemblée par une deuxième partie d'assemblage (51) à un élément d'attache (40),
l'élément d'attache (40) ayant une partie d'attache (41) qui est en mesure d'être attachée à la partie avant au niveau de la taille (30) lors de la mise en place de l'article absorbant,
une résistance d'assemblage de la deuxième partie d'assemblage (51) étant supérieure par rapport à une résistance d'assemblage de la première partie d'assemblage (50), dans lequel
un corps principal absorbant qui constitue la partie au niveau de l'entrejambe (10) est disposé avec sa direction allant dans le sens de la longueur s'étendant le long de la direction allant dans le sens longitudinal,
au niveau de la partie avant au niveau de la taille (30), des ficelles élastiques (33) sont mises en œuvre depuis une ligne centrale (AC) du corps principal absorbant dans la direction allant dans le sens latéral jusqu'à une extrémité de la première partie d'assemblage (50) sur ledit autre côté dans la direction allant dans le sens latéral, et
au niveau de la partie avant au niveau de la taille (30), aucune ficelle électrique n'est mise en œuvre dans une partie à laquelle l'élément d'attache (40) est attaché et qui est située sur ledit autre côté dans la direction allant dans le sens latéral par rapport à la ligne centrale (AC).

2. Article absorbant selon la revendication 1, dans lequel
chacune parmi la première partie d'assemblage (50) et la deuxième partie d'assemblage (51) a une pluralité de régions soudées (52, 53), et
une proportion d'une zone (A2) de la pluralité de régions soudées (53) de la deuxième partie d'assemblage (51) par rapport à une zone (A1) de toute une région de la deuxième partie d'assemblage (51) est supérieure par rapport à une proportion d'une zone (A4) de la pluralité de régions soudées (52) de la première partie d'assemblage (50) par rapport à une zone (A3) de toute une région de la première partie d'assemblage (50).

3. Article absorbant selon la revendication 2, dans lequel
un motif de la pluralité de régions soudées (52) dans la première partie d'assemblage (50) est différent d'un motif de la pluralité de régions soudées (53) dans la deuxième partie d'assemblage (51).

4. Article absorbant selon la revendication 3, dans lequel
une extrémité allant vers l'intérieur dans le sens latéral (53e) de chaque région soudée (53) de la deuxième partie d'assemblage (51) a une longueur prédéterminée le long de la direction allant dans le sens longitudinal.

5. Article absorbant selon la revendication 3 ou la revendication 4, dans lequel
une longueur latérale (d3) de chaque région soudée (52) de la première partie d'assemblage (50) est la plus petite dans au moins l'une ou l'autre parmi une partie d'extrémité supérieure longitudinale de la région soudée (52) et une partie d'extrémité inférieure longitudinale de la région soudée (52).

6. Article absorbant selon l'une quelconque des revendications 1 à 5, dans lequel
alors que la partie d'attache (41) est attachée à la partie avant au niveau de la taille (30) de telle sorte qu'une extrémité de la partie d'attache sur un côté plus proche de la deuxième partie d'assemblage (51) est positionnée dans la direction allant dans le sens latéral sur une extrémité de la partie au niveau de l'entrejambe (10) à l'opposé de la première partie d'assemblage (50),
la deuxième partie d'assemblage (51) est située sur un côté avant par rapport à la première partie d'assemblage (50).

7. Article absorbant selon la revendication 6, dans lequel
une rigidité dans la deuxième partie d'assemblage (51) est supérieure par rapport à une rigidité dans la partie avant au niveau de la taille (30).

8. Article absorbant selon la revendication 7, dans lequel
un certain nombre de composants empilés dans la deuxième partie d'assemblage (51) est supérieur à un certain nombre de composants empilés dans la partie avant au niveau de la taille (30).
